# EUROPÄISCHE PATENTANMELDUNG

(11) **EP 0 564 862 A1**
(43) Veröffentlichungstag der Anmeldung: **13.10.1993**
(21) Anmeldenummer: 93104407.7
(22) Anmeldetag: 18.03.1993
(51) Int. Cl.: C22B 3/00, C22B 11/00, B01J 31/40, C07C 51/573

(54) **Verfahren zur Rückgewinnung von Rhodium aus Rückständen von Carbonylierungsreaktionen**

(30) Priorität: 27.03.1992 DE 4210027
(71) Anmelder: HOECHST AKTIENGESELLSCHAFT, D-65926 Frankfurt am Main (DE)
(72) Erfinder: Lappe, Peter, Dr. Dipl.-Chem., W-4200 Dinslaken (DE); Springer, Helmut, Dipl.-Ing., W-4200 Oberhausen 11 (DE)

(57) **Zusammenfassung**

Die vorliegende Erfindung betrifft ein Verfahren zur Rückgewinnung von Rhodium aus Essigsäureanhydrid enthaltenden Rückständen, die bei der Carbonylierung von Methanol und/oder Methylacetat und/oder Dimethylether anfallen. Man setzt dem Rückstand eine Carbonsäure mit 2 bis 5 Kohlenstoffatomen zu, erwärmt, setzt je Mol Essigsäureanhydrid 1 bis 10 Mol Methanol zu, destilliert die leichter als die Carbonsäure siedenden Bestandteile ab, kühlt ab und separiert den Rhodium enthaltenden Rückstand.

## Beschreibung

Die vorliegende Erfindung betrifft ein Verfahren zur Rückgewinnung von Rhodium aus Rückständen von Carbonylierungsreaktionen. Der Begriff Carbonylierung bezeichnet allgemein die Einführung einer Carbonylgruppe in eine organische Vebindung. Zu den wichtigsten, auch technisch durchgeführten Carbonylierungen, zählen die Synthese von Aldehyden durch Umsetzung von Olefinen mit Kohlenmonoxid und Wasserstoff sowie die Synthese von Carbonsäuren und Carbonsäureanhydriden durch Umsetzung von Alkoholen und/oder Estern und/oder Ethern mit Kohlenmonoxid. Diese Carbonylierungen laufen als Einschiebungsreaktionen üblicherweise nur in Gegenwart von Metallcarbonylen und Carbonylkomplexen ab, die diese Reaktionen katalysieren.

Im Zusammenhang mit der vorliegenden Erfindung wird unter Carbonylierung die Umsetzung von Methanol und/oder Methylacetat und/oder Dimethylether mit Kohlenmonoxid verstanden. Hierbei entstehen Essigsäure und Essigsäureanhydrid. Die Herstellung von Essigsäure aus Methanol und Kohlenmonoxid unter Verwendung von Kobaltcarbonylkatalysatoren zusammen mit einem Jodpromotor einerseits und Rhodiumcarbonylkatalysatoren zusammen mit einem Jodpromotor andererseits ist in Ullmann's Encyclopedia of Industrial Chemistry, 5. Edition, Volume A1 Seiten 47-50, VCH Verlagsgesellschaft Weinheim (1985), beschrieben. Eine zusammenfassende Darstellung der Herstellung von Acetanhydrid durch die Umsetzung von Methylacetat oder Dimethylether in Anwesenheit von Rhodium und Nickelverbindungen, die aktiviert durch Methyljodid, Jodwasserstoff, LiJ, J₂ oder andere Jodide geeignete Katalysatoren darstellen, ist Ullmann's Encyclopedia of Industrial Chemistry, 5. Edition, Volume A1 Seiten 72-74, VCH Verlagsgesellschaft Weinheim (1985), zu entnehmen. Während die Carbonylierung von Methylacetat direkt Essigsäureanhydrid liefert, führt die Umsetzung von Dimethylether mit Kohlenmonoxid zunächst zu Methylacetat, welches anschließend durch erneute Umsetzung mit Kohlenmonoxid zu Acetanhydrid umgewandelt wird.

Die bei der Carbonylierung anfallenden Stoffgemische werden destillativ aufgearbeitet, wobei sich in den durch die Destillation bedingten Rückständen die ursprünglich eingesetzen Carbonylisierungskatalysatoren ansammeln. Die Rückgewinnung von Metallen, insbesondere von Rhodium, aus diesen Rückständen stellt einen wichtigen Teilschritt des Gesamtverfahrens dar.

Die EP 0 210 017 A1 betrifft ein Verfahren zur Rückgewinnung von Rhodium aus Rückständen, die bei der Rhodium-Lithium-katalysierten Carbonylierung von Estern und Ethern in Anwesenheit eines Jodids anfallen. Man behandelt den Rückstand mit einem Alkohol, insbesondere mit einem tertiären Alkohol, wie tert.-Butanol- oder tert.-Amylalkohol, einer Carbonsäure oder einem Carbonsäureester bei erhöhter Temperatur. Hierbei wird ein Feststoff ausgefällt, der den Rhodiumkomplex nahezu vollständig enthält. Zur Abtrennung von teerartigen Bestandteilen wird der ausgefällte Feststoff mit speziellen Lösungsmitteln, beispielsweise Alkanen, Cycloalkanen oder Ethern extrahiert.

Die Verwendung von primären oder sekundären Alkoholen zur Ausfällung des Rhodium enthaltenden Feststoffes führt zu einer signifikant niedrigeren Rhodiumrückgewinnung.

Die EP 0 240 703 betrifft ein Verfahren zur Rhodiumrückgewinnung, wobei zunächst die bei der Carbonylierung von Methylacetat und/oder Dimethylether anfallende, verunreinigte Katalysatorlösung durch eine Flüssig-Flüssig-Extraktion mit Dialkylethern und Alkoholen mit jeweils 1 bis 4 Kohlenstoffatomen von organischen Rückständen befreit wird. Die Etherphase wird anschließend mit Jod und/oder Methyljodid behandelt, der dabei ausfallende Katalysatorkomplex wird abgetrennt und die organische Phase durch Destillation aufgetrennt.

Das Verfahren hat sich bewährt, erfordert jedoch einen vergleichsweise hohen Aufwand.

Die DE 32 08 060 A1 beschreibt ein Verfahren zur Rückgewinnung von Edelmetallen aus Rückständen von Carbonylierungsreaktionen, wobei der Rückstand zunächst in einer ersten Stufe mit einem Alkohol, bevorzugt Methanol behandelt, und dann bei einer Temperatur von nicht über 25°C von Leichtsiedern befreit wird. Dem verbleibenden Rückstand wird ein Amin zugesetzt und das Rhodium durch mehrfache Extraktion mit einer Halogensäure abgetrennt. Das Verfahren erfordert zahlreiche Arbeitsschritte und erfordert bei der unter hohem Vakuum ablaufenden destillativen Abtrennung der Leichtsieder einen hohen technischen Aufwand. Zudem ist die Extraktion mit Halogensäuren wegen der beim Umgang mit Halogensäuren und halogenierten Lösungsmitteln einzuhaltenden Vorschriften mit Nachteilen verbunden.

Es besteht daher die Aufgabe ein Verfahren bereitzustellen, das die vorstehend beschriebenen Nachteile vermeidet, sich technisch in einfacher Weise durchführen läßt, sich üblicher Hilfsstoffe bedient und zudem eine hohe Rückgewinnung von Rhodium sicherstellt.

Gelöst wird diese Aufgabe durch ein Verfahren zur Rückgewinnung von Rhodium aus Essigsäureanhydrid enthaltenden Rückständen, die bei der Carbonylierung von Methanol und/oder Methylacetat und/oder Dimethylether anfallen, dadurch gekennzeichnet, daß man dem Rückstand eine Carbonsäure mit 2 bis 5 Kohlenstoffatomen zusetzt, den mit der Carbonsäure versetzten Rückstand auf 30 bis 100°C erwärmt, mit 1 bis 10 Mol Methanol je Mol Essigsäureanhydrid versetzt, nach Umsetzung des Essigsäureanhydrids mit Methanol die leichter als die Carbonsäure siedenden Bestandteile destillativ entfernt, auf eine Temperatur ≦ 50°C abkühlt und den abgeschiedenen Rhodium enthaltenen Feststoff abtrennt.

Der bei der Carbonylierung von Methanol und/oder Methylacetat und/oder Dimethylether anfallende Rückstand enthält üblicherweise neben untergeordneten Mengen hochsiedender Komponenten als Hauptbestandteile Essigsäure und Essigsäureanhydrid. Der Rückstand enthält 10 bis 60, insbesondere 15 bis 50, bevorzugt 20 bis 40 Gew.-% Essigsäureanhydrid. Ferner weist der Rückstand vergleichsweise niedrige Mengen an Rhodium auf. Er enthält 200 bis 2000, insbesondere 250 bis 1500, bevorzugt 280 bis 1000 ppm Rhodium. Man setzt dem Rückstand eine Carbonsäure in einer Menge zu, daß der Rückstand 10 bis 150, insbesondere 30 bis 120, bevorzugt 50 bis 100 ppm Rhodium enthält.

Geeignete Carbonsäuren sind Essigsäure, Propionsäure, n-Buttersäure, Isobuttersäure, n-Valeriansäure, 2-Methylbuttersäure und 3-Methylbuttersäure. Besonders geeignet als Carbonsäure ist Essigsäure.

Anschließend erwärmt man den mit Carbonsäure versetzten Rückstand auf eine Temperatur von 30 bis 100, insbesondere 35 bis 90, bevorzugt 40 bis 80°C. Anschließend gibt man dem mit Carbonsäure versetzten, erwärmten Rückstand Methanol zu. Die erforderliche Menge Methanol orientiert sich an der im Rückstand vorhandenen Menge Essigsäureanhydrid. Man setzt je Mol Essigsäureanhydrid 1 bis 10 Mol Methanol, insbesondere 1,5 bis 7, bevorzugt 2 bis 6 Mol Methanol zu.

Anschließend läßt man so lange reagieren, bis das Essigsäureanhydrid in ausreichendem Maße umgesetzt ist. Die leichter als die Carbonsäure siedenden Bestandteile, nämlich Essigsäuremethylester und gegebenenfalls überschüssig eingesetztes Methanol werden nachfolgend destillativ abgetrennt. Die Destillation kann bei erhöhter Temperatur in einem Bereich von 50 bis 200 °C durchgeführt werden.

Nach Abtrennung der leichter als die Carbonsäure siedenden Bestandteile wird der Destillationsrückstand auf eine Temperatur ≦ 50, insbesondere ≦ 40, bevorzugt ≦ 35, besonders bevorzugt ≦ 30°C abgekühlt.

Nach Abkühlen fällt ein Rhodium enthalter Niederschlag an, dessen Zusammensetzung nicht näher bekannt ist. Wahrscheinlich handelt es sich um ein Gemisch aus metallischem Rhodium und einem Rhodium-Komplex, der neben Kohlenmonoxid und Jodid noch einen Phosphor enthaltenden Liganden, insbesondere ein Trialkylphosphin, enthält. Dieser Niederschlag kann nach Filtration ebenso wie ein im Reaktionsgefäß verbleibender metallischer Wandbelag durch Behandlung mit Essigsäure und Trialkylphosphin oder Trialkylphospheniumhalogenid unter Zusatz von Kohlenmonoxid in kurzer Zeit bei einer Temperatur von 60 bis 90°C in eine gelöste Form überführt werden. Die resultierende Lösung kann anschließend als Katalysator in den Carbonylierungsprozess zurückgeführt werden.

Das nachfolgende Beispiel belegt die Erfindung, ohne sie zu begrenzen.

### Experimenteller Teil

### Beispiel

50,0 g eines Rückstandes, der 330 ppm Rhodium und 0,131 Mol Essigsäureanhydrid enthält, werden in einem Rundkolben vorgelegt und unter Rühren mit 187,9 g Essigsäure versetzt. Das dabei entstehende, homogene Gemisch wird auf 40°C erwärmt und innerhalb von 15 Minuten mit 8,4 g (0,262 Mol) Methanol versetzt. Man läßt unter Rühren noch 60 Minuten nachreagieren und destilliert anschließend die leichtsiedenden Anteile bis zu einer Temperatur von 109°C (gemessen am Kopf der Destillationseinheit) ab. Es fallen 32,2 g Destillat an. Der Rückstand wird auf 20°C abgekühlt und 20 Stunden gerührt. Anschließend wird der abgeschiedene Rhodium enthaltende Feststoff durch Filtration separiert. In der anfallenden Mutterlauge sind noch 1,33 mg Rhodium, d. h. 8,1 Gew.-% des ursprünglich eingesetzten Rhodiums homogen gelöst.

Der abfiltrierte Feststoff wird in den Rundkolben zurückgeführt, mit 60,0 g Essigsäure sowie mit 6,0 g Methyl-tri-n-butylphosphoniumjodid versetzt und 60 Minuten bei 80°C unter Durchleiten von Kohlenmonoxid gerührt. Es fällt eine klare, homogene Lösung mit einem Rhodiumgehalt von 14,65 mg, entsprechend 88,8 Gew.-% der ursprünglich eingesetzten Rhodiummenge, an.

## Patentansprüche

1. Verfahren zur Rückgewinnung von Rhodium aus Essigsäureanhydrid enthaltenden Rückständen, die bei der Carbonylierung von Methanol und/oder Methylacetat und/oder Dimethylether anfallen, dadurch gekennzeichnet, daß man dem Rückstand eine Carbonsäure mit 2 bis 5 Kohlenstoffatomen zusetzt, den mit der Carbonsäure versetzten Rückstand auf 30 bis 100°C erwärmt, mit 1 bis 10 Mol Methanol je Mol Essigsäureanhydrid versetzt, nach Umsetzung des Essigsäureanhydrids mit Methanol die leichter als die Carbonsäure siedenden Bestandteile destillativ entfernt, auf eine Temperatur ≦ 50°C abkühlt und den abgeschiedenen, Rhodium enthaltenen Feststoff abtrennt.

2. Verfahren nach Anspruch 1, dadurch gekennzeichnet, daß der Rückstand 10 bis 60, insbesondere 15 bis 50, bevorzugt 20 bis 40 Gew.-% Essigsäureanhydrid enthält.

3. Verfahren nach Anspruch 1 oder 2, dadurch gekennzeichnet, daß der Rückstand 200 bis 2000, insbesondere 250 bis 1500, bevorzugt 280 bis 1000 ppm Rhodium enthält.

4. Verfahren nach einem oder mehreren der Ansprüche 1 bis 3, dadurch gekennzeichnet, daß man dem Rückstand soviel Carbonsäure zusetzt, daß der Rückstand 10 bis 150, insbesondere 30 bis 120, bevorzugt 50 bis 100 ppm Rhodium enthält.

5. Verfahren nach einem oder mehreren der Ansprüche 1 bis 4, dadurch gekennzeichnet, daß man als Carbonsäure Essigsäure einsetzt.

6. Verfahren nach einem oder mehreren der Ansprüche 1 bis 5, dadurch gekennzeichnet, daß der mit der Carbonsäure versetzte Rückstand auf eine Temperatur von 35 bis 90, insbesondere 40 bis 80°C erwärmt wird.

7. Verfahren nach einem oder mehreren der Ansprüche 1 bis 6, dadurch gekennzeichnet, daß man je Mol Essigsäureanhydrid 1,5 bis 7, insbesondere 2 bis 6 Mol Methanol zusetzt.

8. Verfahren nach einem oder mehreren der Ansprüche 1 bis 7, dadurch gekennzeichnet, daß die leichter als die Carbonsäure siedenden Bestandteile Essigsäuremethylester und gegebenenfalls überschüssig eingesetztes Methanol destillativ abgetrennt werden.

9. Verfahren nach einem oder mehreren der Ansprüche 1 bis 8, dadurch gekennzeichnet, daß man nach Abtrennung der leichter als die Carbonsäure siedenden Bestandteile auf eine Temperatur ≦ 40, insbesondere ≦ 35, bevorzugt ≦ 30°C abkühlt.
